# EUROPÄISCHE PATENTANMELDUNG

(11) **EP 4 470 606 A1**
(43) Veröffentlichungstag der Anmeldung: **04.12.2024**
(21) Anmeldenummer: 23176055.4
(22) Anmeldetag: 30.05.2023
(51) Int. Cl.: A61N 2/00

(54) **APPLIKATOREINRICHTUNG ZUM APPLIZIEREN VON MAGNETISCHEN FELDERN ZUR MAGNETFELDTHERAPIE**

(71) Anmelder: Gbo Medizintechnik AG, 64668 Rimbach (DE)
(72) Erfinder: KEßLER, Michael, 69483 Wald-Michelbach (DE)
(74) Vertreter: Franke, Markus

(57) **Zusammenfassung**

Die Erfindung betrifft eine Applikatoreinrichtung (1) zum Applizieren von magnetischen Feldern zur Magnetfeldtherapie mit mehreren Applikatoren zum Applizieren von magnetischen Feldern. Die die Applikatoreinrichtung (1) weist einen elastisch verformbaren Tragbügel (2) mit einem ersten Schenkel (3a), einem zweite Schenkel (3b) und mit einem den ersten Schenkel (3a) mit dem zweiten Schenkel (3b) verbindenden Verbindungsabschnitt (4) auf, wobei der Tragbügel (2) dazu angepasst ist, bei Verwendung der Applikatoreinrichtung (1) im Kopfbereich einer Person getragen zu werden, derart, dass der Verbindungsabschnitt (4) sich über das Gesicht der Person, insbesondere die Augen der Person erstreckt, und sich die Schenkel (3a, 3b) in Richtung der Ohren erstrecken, wobei in oder an dem Verbindungsabschnitt (4) ein Paar von Augenapplikatoren und in oder an den Schenkeln (3a, 3b) jeweils ein Ohrenapplikator (5a, 5b) angeordnet sind, wobei der jeweilige Applikator eine Spule (6) aufweist zur Erzeugung eines magnetischen Feldes bei einem Stromfluss durch die Spule (6).

## Beschreibung

Die Erfindung betrifft eine Applikatoreinrichtung zum Applizieren von magnetischen Feldern zur Magnetfeldtherapie mit mehreren Applikatoren zum Applizieren von magnetischen Feldern.

Magnetfeldtherapien, bei denen ein Organismus, insbesondere ein menschlicher Organismus, mit einem zeitlich variierendem Magnetfeld beaufschlagt wird, werden heutzutage in vielen Bereichen angewendet, um das Wohlbefinden und die Gesundheit zu fördern. Die Erzeugung der, in der Regel niederfrequenten, magnetischen Felder erfolgt in der Regel über stromdurchflossene Spulen. Beispielsweise wird in der DE 10 2018 119 019 A1 eine Vorrichtung zum Aussenden von elektromagnetischen Feldern beschrieben, wobei diese Vorrichtung zwei Aktuatoren zum Aussenden von elektromagnetischen Feldern aufweist, wobei diese beiden Aktuatoren dazu eingerichtet sind, jeweils elektromagnetische Felder mit unterschiedlicher Frequenz auszusenden. Aus dem Stand der Technik sind Vorrichtungen bekannt, die dazu dienen, den Augenbereich einer zu behandelnden Person mit einem Magnetfeld zu beaufschlagen. Beispielsweise wird in der WO 2010/022419 A1 eine Vorrichtung zur Erzeugung eines elektromagnetischen Felds mit einer Frequenz im Bereich der Schwingungsfrequenz des Erdmagnetfelds beschrieben, wobei eine elektrische Leitung zum Aufbau des elektromagnetischen Feldes in einer Brille angeordnet ist, sodass in der Gebrauchslage der Brille das elektromagnetische Feld im Augenbereich der zu behandelnden Person erzeugt wird. Die US 2019/0299020 A1, die WO 2020/206032 A1 und die WO 2019/173866 A1 beschreiben weiteren Stand der Technik.

Neben Applikatoren, die zur Behandlung von Augen einer Person mit einem Magnetfeld dienen, sind auch Applikatoren bekannt, die zur Behandlung von Ohren einer Person mit elektromagnetischen Feldern dienen.

Die gleichzeitige Behandlung von den Ohren einer Person und den Augen einer Person mit Magnetfeldern ist in der Regel nicht möglich. Es sind zwar Magnetfeldtherapiegeräte bekannt, die eine separate Signalerzeugungseinheit aufweisen, wobei an die Signalerzeugungseinheit unterschiedliche Applikatoren angeschlossen werden können, wobei die Signalerzeugungseinheit dazu dient, das Stromsignal für die stromdurchflossenen Spulen des an das Gerät angeschlossenen Applikators zu erzeugen. Allerdings kann bei derartigen Magnetfeldtherapiegeräten lediglich einer der beiden Applikatoren, nämlich entweder Augenapplikator oder der Ohrenapplikator, an die Signalerzeugungseinheit angeschlossen werden. Zudem sind die Applikatoren häufig von ihrer Gestaltung bzw. Anatomie her nicht dazu geeignet, gemeinsam verwendet zu werden. Dies insbesondere, da Applikatoren für die Augen häufig in Form einer Brille ausgebildet sind, deren Brillenbügel auf und hinter den Ohren anliegen, sodass die zusätzliche Anordnung eines in Art eines Kopfhörers ausgebildeten Magnetfeldtherapiegeräts bzw. Applikators für die Ohren bei kombinierter Anwendung mit dem Augenapplikator häufig für die zu behandelnde Person unangenehm ist.

Es besteht daher Bedarf an einer Applikatoreinrichtung, die sowohl für die Behandlung der Augen als auch für die Behandlung der Ohren und insbesondere zur kombinierten Behandlung von Augen und Ohren geeignet ist.

Gelöst wird diese Aufgabe durch eine Applikatoreinrichtung, die die Merkmale des Anspruchs 1 aufweist. Vorteilhafte Weiterbildungen sind Gegenstand der abhängigen Ansprüche.

Die erfindungsgemäße Applikatoreinrichtung dient zur Behandlung der Augen einer Person und zur Behandlung der Ohren einer Person und insbesondere zur kombinierten Behandlung der Augen und Ohren einer Person. Zu diesem Zweck weist die Applikatoreinrichtung mehrere Applikatoren zum Applizieren von magnetischen Feldern auf, insbesondere zum Applizieren von zeitlich veränderlichen magnetischen Feldern, insbesondere zur Applikation von elektromagnetischen Feldern. Die Applikatoreinrichtung weist einen elastisch verformbaren Tragbügel mit einem ersten Schenkel, einem zweiten Schenkel und einem den ersten Schenkel mit dem zweiten Schenkel verbindenden Verbindungsabschnitt auf, wobei der Tragbügel dazu angepasst ist, bei Verwendung der Applikatoreinrichtung, somit in der Gebrauchslage, im Kopfbereich einer Person getragen zu werden, derart, dass der Verbindungsabschnitt sich über das Gesicht der Person, insbesondere die Augen der Person erstreckt, und sich die Schenkel in Richtung der Ohren erstrecken, wobei in oder an dem Verbindungsabschnitt ein Paar von Augenapplikatoren und in oder an den Schenkeln jeweils ein Ohrenapplikator angeordnet sind, wobei der jeweilige Applikator eine Spule aufweist zur Erzeugung eines magnetischen Feldes bei einem Stromfluss durch die Spule. Das Paar von Augenapplikatoren dient somit zur Behandlung der Augen der Person und die Ohrenapplikatoren dienen zur Behandlung der Ohren der Person.

Die Applikatoreinrichtung ermöglicht durch diese Gestaltung einen hohen Tragekomfort für die zu behandelnde Person und die Behandlung der Augen, der Ohren oder die gleichzeitige Behandlung der Augen und Ohren der Person. Dementsprechend ist es nicht mehr notwendig, zwei unterschiedliche Magnetfeldtherapiegeräte bzw. separate Applikatoren, nämlich ein Magnetfeldtherapiegerät bzw. einen Applilator für die Augen und ein weiteres Magnetfeldtherapiegerät bzw. einen weiteren Applikator für die Ohren vorzusehen, sondern lediglich nur noch eine einzige, nämlich die erfindungsgemäße kombinierte Applikatoreinrichtung für diese Anwendungen vorzusehen. Dadurch, dass der Tragbügel elastisch verformbar ist, passt sich der Tragbügel optimal an die Abmessungen des Kopfes der zu behandelnden Person an und gewährleistet einen guten Halt der Applikatoreinrichtung auch ohne weitere Haltemittel, da die Schenkel aufgrund der Rückstellkräfte des elastisch verformbaren Tragbügels einen guten Halt an dem Kopf der zu behandelnden Person gewährleistet. Aufgrund der Gestaltung mit dem elastisch verformbaren Tragbügel ist es daher nicht notwendig, weitere Befestigungs- oder Haltemittel an der Applikatoreinrichtung vorzusehen.

Eine Spule im Sinne der vorliegenden Erfindung umfasst zumindest einen Leiter der zumindest eine Wicklung bzw. Windung aufweist. Bei der jeweiligen Spule kann es sich beispielsweise um eine Flachspule, eine Schneckenspule, eine Luftspule oder eine Ringspule handeln. Vorzugweise weist die jeweilige Spule zumindest 30 Windungen auf. Bei dem Leiter kann es sich beispielsweise um einen Kupferdraht handeln. Vorzugsweise besteht die jeweilige Spule aus einem einzigen, gewickelten Leiter. Vorzugsweise weisen die Spulen der Ohrenapplikatoren eine größere Anzahl von Wicklungen auf als die Spulen der Augenapplikatoren.

Vorzugsweise stützt sich der Verbindungsabschnitt in der Gebrauchslage an der Nase der zu behandelnden Person ab. Dadurch ist zudem gewährleistet, dass die Augenapplikatoren in definierter Position zu den Augen der zu behandelnden Person stehen und der Halt der Applikatoreinrichtung wird verbessert.

Es wird als besonders vorteilhaft angesehen, wenn die Schenkel zur Verwendung im Kopfbereich der Person entgegen einer Rückstellkraft auseinandergebogen werden müssen, wodurch ein besonders guter Halt im Kopfbereich erreicht wird. In diesem Zusammenhang wird es als besonders vorteilhaft angesehen, wenn die Schenkel in einem Ausgangszustand der Applikatoreinrichtung aufeinander zu gerichtet sind, beispielsweise um mehr als 190° umgebogen sind, und zur Verwendung der Applikatoreinrichtung auseinandergebogen werden müssen.

Es wird ferner als vorteilhaft angesehen, wenn der Tragbügel U-förmig gebogen ausgebildet ist, insbesondere die gesamte Applikatoreinrichtung im Wesentlichen U-förmig ausgebildet ist, somit keine weiteren Befestigungskomponenten oder Tragekomponenten, wie beispielsweise ein Kopfbügel, Befestigungsband oder Gummiband, vorgesehen sind.

Es wird als besonders vorteilhaft angesehen, wenn die Spulen der Augenapplikatoren ortsfest in dem Tragbügel angeordnet sind. Es hat sich in der Praxis gezeigt, dass die Stellung der Augen zueinander zwischen verschiedenen Personen nicht derart stark variiert, dass es notwendig ist, die Spulen hinsichtlich ihrer Anordnung in dem Tragbügel anzupassen. Dementsprechend kann auch mit ortsfesten Spulen bei unterschiedlichen Personen die gewünschte Magnetfeldbehandlung im Augenbereich erreicht werden. Dadurch wird der Aufbau der Applikatoreinrichtung erheblich erleichtert, wodurch auch Gewicht eingespart werden kann, was sich vorteilhaft auf den Tragekomfort der Applikatoreinrichtung auswirkt.

Es wird als vorteilhaft angesehen, wenn der erste Ohrenapplikator im Bereich eines freien Endes des ersten Schenkels und der zweite Ohrenapplikator im Bereich eines freien Endes des zweiten Schenkels angeordnet sind.

In einer bevorzugten Weiterbildung ist vorgesehen, dass der jeweilige Ohrenapplikator entlang des jeweiligen Schenkels verschiebbar in dem jeweiligen Schenkel gelagert ist. Es hat sich in der Praxis gezeigt, dass durch das Verstellen der beiden Ohrenapplikatoren entlang des jeweiligen Schenkels eine sehr gute Anpassung der Applikatoreinrichtung an die Anatomie des Kopfes der jeweiligen Person erreicht werden kann. Dabei hat sich gezeigt, dass eine Verschiebbarkeit entlang des jeweiligen Schenkels ausreichend ist, um eine ausreichende Anpassung der Applikatoreinrichtung bzw. der Anordnung der Applikatoren zu erreichen. Beispielsweise hat sich gezeigt, dass es nicht notwendig ist, in weiteren Raumrichtungen eine Verstellung der Ohrenapplikatoren bezogen auf die Augenapplikatoren vorzunehmen. Dementsprechend wird es als besonders vorteilhaft angesehen, wenn der jeweilige Ohrenapplikator lediglich entlang des jeweiligen Schenkels verschiebbar ist, wodurch wiederum ein besonders einfacher Aufbau der Applikatoreinrichtung erreicht werden und zudem Gewicht eingespart werden kann, was sich wiederum vorteilhaft auf den Tragekomfort auswirkt.

Es wird als besonders vorteilhaft angesehen, wenn die Schenkel einteilig mit dem Verbindungsabschnitt ausgebildet sind. Dementsprechend sind die Schenkel vorzugsweise scharnierlos mit dem Verbindungabschnitt verbunden.

Vorzugsweise besteht der Tragbügel aus einem elastisch verformbaren Material, insbesondere aus einem Kunststoff, vorzugsweise aus Acrylnitril-Butadien-Styrol (ABS).

Es wird als besonders vorteilhaft angesehen, wenn in dem Tragbügel ein Aufnahmebereich für die Spulen der Augenapplikatoren vorgesehen ist sowie ein Kanal zur Anordnung der notwendigen elektrischen Verkabelung, wobei die elektrische Verkabelung jeweils bis zu den Schenkeln des Tragbügels und von dort zu den Ohrenapplikatoren geführt ist.

Vorzugweise bildet der Tragbügel ein Gehäuseschale mit einem Aufnahmeraum zur Aufnahme der Spulen der Augenapplikatoren und/oder einem Aufnahmeraum für die Verkabelung, wobei die Gehäuseschale mit einem korrespondierenden Gehäusedeckel verschlossen ist, wodurch die Verkabelung und/oder die Spulen der Augenapplikatoren vor äußeren Einflüssen geschützt sind. Der Gehäusedeckel ist vorzugsweise mit der Gehäuseschale verklipst. Diesbezüglich wird es als besonders vorteilhaft angesehen, wenn der Deckel ebenfalls U-förmig gebogen ausgebildet ist. Der Deckel besteht vorzugsweise ebenfalls aus einem elastischen Material, insbesondere aus ABS.

In einer vorteilhaften Weiterbildung ist vorgesehen, dass der jeweilige Ohrenapplikator ein Gehäuse umfasst, wobei in dem Gehäuse die Spule des jeweiligen Ohrenapplikators aufgenommen ist. Im Bereich des Ohrenapplikators können auch weitere Komponenten der Applikatoreinrichtung angeordnet oder angebracht sein. Beispielsweise wird es als besonders vorteilhaft angesehen, wenn eine elektrische Anbindung an eine externe Stromversorgung, insbesondere in Form eines Kabels, im Bereich eines oder beider Ohrenapplikatoren angebracht ist. Dies deshalb, da die Verkabelung in diesem Bereich für die zu behandelnde Person am wenigsten störend ist und zudem auch Zugkräfte oder Gewichtskräfte im Bereich der Ohrenapplikatoren hinsichtlich des Tragekomforts vorteilhafter sind, als dies der Fall wäre, wenn die Verkabelung im Bereich des Verbindungsabschnitts angebracht wäre.

Zur Erhöhung des Tragekomforts der Applikatoreinrichtung wird es als besonders vorteilhaft angesehen, wenn der Verbindungsabschnitt in seinem auf der Nase aufliegenden Bereich eine an die Nasenform angepasste Struktur, beispielsweise in Form einer Ausnehmung aufweist. Es ist aber auch denkbar, dass an dem Verbindungsabschnitt an separates Nasenpad oder ein Nasensteg angebracht ist, wie dies beispielweise von herkömmlichen Brillen bekannt ist. Vorzugsweise weist der Verbindungsabschnitt in dem an der Nase zur Anlage kommenden Bereich eine Ausnehmung auf.

Es wird als besonders vorteilhaft angesehen, wenn der Verbindungsabschnitt eine Sichtöffnung für das jeweilige Auge aufweist. Bei Verwendung der Applikatoreinrichtung kann somit die Person dennoch das Umfeld beobachten, lesen oder sonstige Tätigkeiten mit den Augen verrichten. Als besonders vorteilhaft in diesem Zusammenhang wird es angesehen, wenn die Spulen der Augenapplikatoren als Hohlspulen oder Ringspulen ausgebildet sind, somit diese Spulen umlaufend um die Sichtöffnung angeordnet werden können. Dadurch ist eine optimale Applikation des Magnetfelds im Bereich des jeweiligen Auges möglich und die Person kann dennoch durch den hohlen Kern der Spule und die vorgesehene Sichtöffnung hindurchblicken.

Vor dem Hintergrund einer möglichst nahen Anordnung der Spulen der Augenapplikatoren an den Augen der zu behandelnden Person wird es als besonders vorteilhaft angesehen, wenn der Verbindungsabschnitt zwei Aufnahmetaschen aufweist, wobei in den Aufnahmetaschen die Spule des jeweiligen Augenapplikators aufgenommen sind, wobei die jeweilige Aufnahmetasche als eine in Richtung einer der Person zuzuwendenden Rückseite des Verbindungsabschnitts hervorstehende Ausbuchtung ausgebildet ist. Somit ist die Rückseite der Aufnahmetasche geschlossen und dadurch die Spule verdeckt.

Dabei wird es als besonders vorteilhaft angesehen, wenn der Verbindungsabschnitt einen dem jeweiligen Augenapplikator zugeordneten, hohlzylindrischen Lagerabschnitt aufweist, wobei die Spule des jeweiligen Augenapplikators auf den entsprechenden Lagerabschnitt aufgeschoben oder herumgewickelt ist zum radialen Lagern der Spule. Vorzugsweise ist der jeweilige Lagerabschnitt durch einen ringförmigen, in Richtung der Frontseite hervorstehenden Vorsprung gebildet. Der hohlzylindrischen Lagerabschnitt weist vorzugsweise einen ovalen Querschnitt auf.

Es wird als besonders vorteilhaft angesehen, wenn an dem Verbindungsabschnitt auf einer der Person abzuwendenden Frontseite ein den Verbindungsabschnitt abdeckendes Verkleidungselement angebracht ist, wobei das Verkleidungselement an dem Verbindungsabschnitt befestigt ist und die Schenkel unabhängig von dem Verkleidungselement elastisch verformbar sind, insbesondere derart, dass ein Auseinanderbiegen der Schenkel bis zu einer Maximalweite ermöglicht ist, wobei das Verkleidungselement ein weiteres Aufbiegen über die Maximalweite verhindert. Diese Gestaltung hat den Vorteil, dass das Verkleidungselement weitestgehend unabhängig von dem Tragbügel, der elastisch verformbar ist, ausgebildet sein kann, ohne die Funktionalität des elastisch verformbaren Tragbügels hinsichtlich eines optimalen Anformens an den Kopfbereich der Person zu beeinträchtigen. Dementsprechend kann das Verkleidungselement weitestgehend unter Designaspekten und insbesondere auch formstabil bzw. steif gestaltet werden, ohne auf funktionale Aspekte hinsichtlich der Anformung an den Kopf der Person Rücksicht zu nehmen, da die funktionalen Aspekte von dem elastisch verformbaren Tragbügel bereitgestellt werden. Dadurch, dass das Verkleidungselement, vorzugsweise ausschließlich, an dem Verbindungsabschnitt befestigt ist, wird eine Funktionalität der elastisch verformbaren Schenkel durch das Verkleidungselement nicht beeinträchtigt. Dementsprechend kann das Verkleidungselement in einer bevorzugten Ausführungsform formstabiler sein als der Tragbügel, beispielsweise aus einem steiferen Material bestehen. Das Verkleidungselement kann den Deckel für die Gehäuseschale bilden.

Vorzugsweise ist das Verkleidungselement in einem zentralen Bereich des Verbindungsabschnitts an diesem befestigt, beispielsweise mit diesem verschraubt. Vorzugweise erfolgt die Verschraubung von der Rückseite aus, sodass die Verschraubung von der Frontseite aus nicht sichtbar ist.

Ferner wird es als besonders vorteilhaft angesehen, wenn das Verkleidungselement zumindest im Bereich der Sichtöffnungen durchsichtig ist. Vorzugsweise ist das Verkleidungselement aus einem durchsichtigen Material gefertigt.

Bei dem Material des Verkleidungselements kann es sich beispielsweise um ein Glas, insbesondere um ein Kunststoffglas, wie beispielsweise Acrylglas handeln. Vorzugsweise handelt es sich bei dem Material des Verkleidungselements um transparentes ABS.

Zudem besteht auch die Möglichkeit, dass das Verkleidungselement im Bereich der Sichtöffnungen mit einer gewissen Brillenstärke versehen ist, um einen Dioptrieausgleich bei einer Sehschwäche der zu behandelnden Person zu kompensieren. Dadurch wird es der die Applikatoreinrichtung tragenden Person ermöglicht, trotz einer Sehschwäche während der Therapie zu lesen, fernzuschauen oder sonstige Tätigkeiten mit dem Auge durchzuführen. Durch Vorhalten von unterschiedlichen Verkleidungselementen kann ein zu der Sehschwäche der behandelnden Person passendes Verkleidungselement ausgewählt und an den Verbindungsabschnitt angebracht werden. Eine optimale Anpassung ist in der Regel nicht erforderlich, wie dies beispielsweise auch von Lesebrillen bekannt ist.

Es ist durchaus denkbar, dass das Verkleidungselement lösbar an dem Verbindungsabschnitt angebracht ist. Dadurch wird ein einfacher Austausch des Verkleidungselements ermöglicht. Dadurch kann eine einfache Anpassung des Designs der Applikatoreinrichtung und/oder bei geeigneten Verkleidungselementen eine Anpassung an die Sehschwäche bzw. Fehlsichtigkeit der Person erfolgen.

Es wird als besonders vorteilhaft angesehen, wenn das Verkleidungselement auf der der Person abzuwendenden Außenseite verspiegelt ist.

Da in der Regel zur Behandlung der Augen und zur Behandlung der Ohren bei der Magnetfeldtherapie unterschiedliche Magnetfelder eingesetzt werden, das heißt, Magnetfelder eingesetzt werden, die sich beispielsweise hinsichtlich ihrer Frequenz und/oder ihrer Stärke und/oder ihrer Pulsweite und/oder ihrer Pulsfrequenz unterscheiden, wird es als besonders vorteilhaft angesehen, wenn die Applikatoreinrichtung dazu eingerichtet ist, die Ohrenapplikatoren und die Augenapplikatoren unterschiedlich anzusteuern, das heißt das die jeweilige Spule durchfließende Stromsignal unterschiedlich zu gestalten. Dementsprechend wird es als besonders vorteilhaft angesehen, wenn die Applikatoreinrichtung für die Ohrenapplikatoren und die Augenapplikatoren jeweils über separate Signalerzeugungseinheiten ansteuerbar sind oder separate Anschlüsse aufweisen. Dementsprechend sind die Spulen der Augenapplikatoren stromsignaltechnisch von den Spulen der Ohrenapplikatoren getrennt. Hingegen sind die Spulen der Ohrenapplikatoren vorzugsweise stromsignaltechnisch miteinander verbunden, sodass von der jeweiligen Spule das gleiche Magnetfeld erzeugt wird. Entsprechendes gilt für die Spulen der Augenapplikatoren.

Vorzugsweise sind die Signalerzeugungseinheiten für die Applikatoren in der Applikatoreinrichtung integriert, sodass auf eine separate, beispielsweise stationäre, Signalerzeugungseinheit verzichtet werden kann. Die Signalerzeugungseinheit wird somit gemeinsam mit der Signalerzeugungseinheit am Kopf getragen. Es wird dann lediglich noch eine Stromquelle benötigt, vorzugsweise eine Gleichstromquelle, die elektrisch mit der jeweiligen Signalerzeugungseinheit verbunden ist. Bei der Stromquelle kann es sich um ein separates Gerät handeln, dass über ein Verbindungskabel mit der Applikatoreinrichtung verbunden ist. Grundsätzlich sind aber auch Batterien oder Akkumulatoren, beispielsweise in Form einer Lithium-Ionen-Batterie, als Stromquelle denkbar. Dadurch wird eine möglichst autarke Verwendung der Applikatoreinrichtung ermöglicht.

Es wird als besonders vorteilhaft angesehen, wenn die Applikatoreinrichtung eine erste Signalerzeugungseinheit für den jeweiligen Augenapplikator oder eine gemeinsame erste Signalerzeugungseinheit für beide Augenapplikatoren aufweist, wobei die ersten Signalerzeugungseinheiten oder die gemeinsame erste Signalerzeugungseinheit mit den Spulen der Augenapplikatoren elektrisch verbunden sind, wobei die ersten Signalerzeugungseinheiten oder die gemeinsame erste Signalerzeugungseinheit dazu eingerichtet sind, dass die Spulen der Augenapplikatoren durchfließenden Stromsignal zu erzeugen, wobei die ersten Signalerzeugungseinheiten oder die gemeinsame erste Signalerzeugungseinheit in einem der Ohrenapplikatoren integriert sind. Dies hat den wiederum den Vorteil, das Gewicht im Bereich der Verbindungsabschnitte eingespart wird und dennoch die entsprechende Signalerzeugungseinheit unmittelbar in der Applikatoreinrichtung integriert ist, sodass auf eine externe Signalerzeugungseinheit verzichtet werden kann. Dadurch wird eine möglichst autarke Verwendung der Applikatoreinrichtung ermöglicht. So ist es durchaus denkbar, dass die Applikatoreinrichtung lediglich noch mit einer Stromquelle verbunden werden muss, da die Signalformung bzw. Signalerzeugung für die stromdurchflossenen Spulen in der Applikatoreinrichtung erfolgt. Dadurch eröffnet sich auch die Möglichkeit, die Applikatoreinrichtung möglichst einfach anzusteuern, um die gewünschte Signalform zu erzeugen, wobei diese Ansteuerung beispielsweise mit einem mobilen Gerät, insbesondere einem Mobiltelefon, erfolgen kann. Vor diesem Hintergrund wird es ebenfalls als besonders vorteilhaft angesehen, wenn die Applikatoreinrichtung eine Kommunikationsschnittstelle aufweist, die eine Kommunikationsverbindung mit einem mobilen Endgerät ermöglicht. Diese Kommunikationsschnittstelle ist vorzugsweise als drahtlose Kommunikationsschnittstelle ausgeführt.

Die jeweilige erste Signalerzeugungseinheit kann auch eine Speichereinrichtung umfassen, in der voreingestellte Therapieprogramme und entsprechende Stromsignalformen hinterlegt sind.

Hinsichtlich der ersten Signalerzeugungseinheiten bzw. der gemeinsamen ersten Signalerzeugungseinheit wird es als besonders vorteilhaft angesehen, wenn diese Signalerzeugungseinheit innerhalb eines Gehäuses des jeweiligen Ohrenapplikators ausgebildet ist.

Es wird als besonders vorteilhaft angesehen, wenn die Applikatoreinrichtung eine zweite Signalerzeugungseinheit für den jeweiligen Ohrenapplikator oder eine gemeinsame zweite Signalerzeugungseinheit für beide Ohrenapplikatoren aufweist, wobei die zweiten Signalerzeugungseinheiten oder die gemeinsame zweite Signalerzeugungseinheit mit den Spulen der Ohrenapplikatoren elektrisch verbunden sind, wobei die zweiten Signalerzeugungseinheiten oder die gemeinsame zweite Signalerzeugungseinheit dazu eingerichtet ist, dass die Spulen der Ohrenapplikatoren durchfließende Stromsignal zu erzeugen, wobei die zweiten Signalerzeugungseinheiten oder die gemeinsame zweite Signalerzeugungseinheit in einem der Ohrenapplikator integriert sind.

Die zu der ersten Signalerzeugungseinheit genannten Vorteile und vorteilhaften Weiterbildungen gelten entsprechend für die zweite Signalerzeugungseinheit.

Es wird als besonders vorteilhaft angesehen, wenn in dem jeweiligen Ohrenapplikator zumindest eine der Signalerzeugungseinheiten integriert ist, insbesondere in dem einen Ohrenapplikator die gemeinsame erste Signalerzeugungseinheit und in dem anderen Ohrenapplikator die gemeinsame zweite Signalerzeugungseinheit integriert ist. Dadurch sind die unterschiedlichen Signalerzeugungseinheiten räumlich voneinander getrennt, wodurch eine etwaige Wechselwirkung zwischen den Signalerzeugungseinheiten, die sich ggf. negativ auf das entsprechende Stromsignal auswirken könnten, vermieden oder zumindest abgeschwächt wird.

Es wird als besonders vorteilhaft angesehen, wenn die jeweilige Signalerzeugungseinheit eine Leiterplatte mit darauf angebrachten Elektronikkomponenten umfasst. Dadurch kann die jeweilige Signalerzeugungseinheit besonders einfach in dem jeweiligen Ohrenapplikator, insbesondere in dem jeweiligen Gehäuse, montiert werden.

Es wird als besonders vorteilhaft angesehen, wenn die Spule des jeweiligen Ohrenapplikators unmittelbar an die in dem entsprechenden Ohrenapplikator ausgebildete Signalerzeugungseinheit angelötet ist.

Die Applikatoreinrichtung ist insbesondere dazu eingerichtet, im Bereich des jeweiligen Applikators ein Magnetfeld zu erzeugen, wobei ein Effektivwert dieses Magnetfelds zwischen 40 µT und 1 mT beträgt.

Vorzugsweise ist die Applikatoreinrichtung dazu eingerichtet, ein Magnetfeld mit einer Pulsfrequenz von kleiner oder gleich 500 Hz zu erzeugen.

In den nachfolgenden Figuren wird die Erfindung anhand von Ausführungsbeispielen näher erläutert, ohne auf diese beschränkt zu sein. Es zeigen:
- Fig. 1: eine erste Ausführungsform einer Applikatoreinrichtung zur Behandlung von Augen und Ohren einer Person in einer Ansicht von schräg vorne,
- Fig. 2: die Applikatoreinrichtung gemäß Fig. 1 ohne frontseitiges Verkleidungselement,
- Fig. 3: die Applikatoreinrichtung gemäß Fig. 1 in einer Seitenansicht,
- Fig. 4: die Applikatoreinrichtung gemäß Fig. 1 in einer Seitenansicht ohne frontseitiges Verkleidungselement,
- Fig. 5: die Applikatoreinrichtung gemäß Fig. 1 in einer Frontansicht,
- Fig. 6: die Applikatoreinrichtung gemäß Fig. 1 in einer Frontansicht ohne frontseitiges Verkleidungselement,
- Fig. 7: die Applikatoreinrichtung gemäß Fig. 1 in einer teilweise geschnittenen Darstellung in einer in einer Ansicht von schräg hinten,
- Fig. 8: ein Ausschnitt der Fig. 7 in einer vergrößerten Darstellung,
- Fig. 9: eine zweite Ausführungsform der Applikatoreinrichtung in einer Ansicht von schräg vorne.

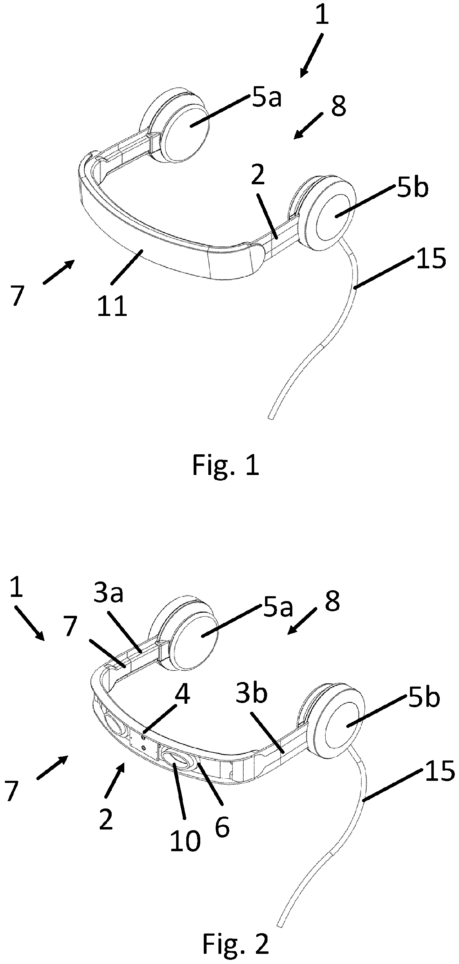

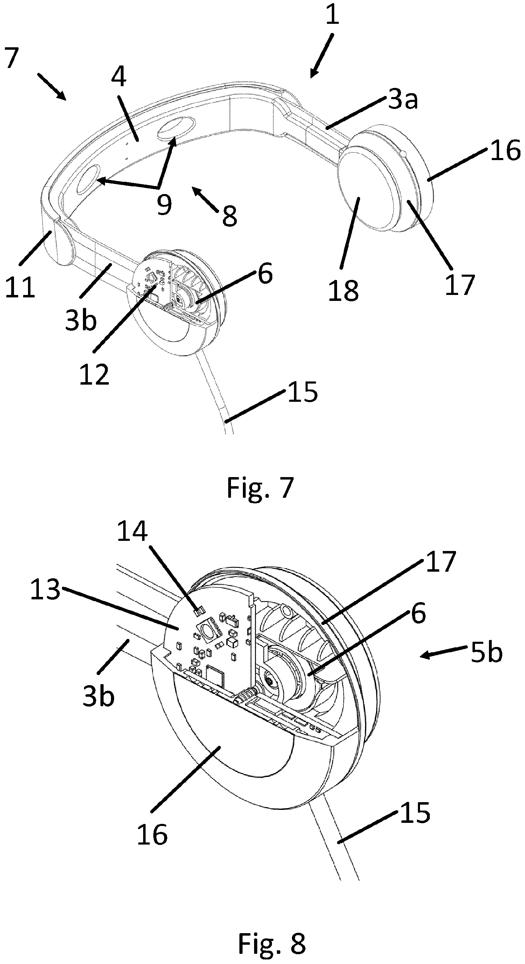

Die Fig. 1 bis 8 zeigen eine erste Ausführungsform einer Applikatoreinrichtung 1, wobei diese Applikatoreinrichtung 1 dazu eingerichtet ist, sowohl im Bereich der Augen einer zu behandelnden Person als auch im Bereich der Ohren einer zu behandelnden Person ein Magnetfeld zu applizieren. Die Applikatoreinrichtung 1 ist dazu eingerichtet, im Kopfbereich einer zu behandelnden Person getragen zu werden. Zu diesem Zweck weist die Applikatoreinrichtung 1 einen elastisch verformbaren Tragbügel 2 mit einem ersten Schenkel 3a, einem zweiten Schenkel 3b und einen den ersten Schenkel 3a mit dem zweiten Schenkel 3b verbindenden Verbindungsabschnitt 4 auf. Der Tragbügel 2 als solcher ist U-förmig ausgebildet und dazu angepasst, bei Verwendung der Applikatoreinrichtung 1, insofern in der Gebrauchslage, im Kopfbereich einer Person getragen zu werden, derart, dass der Verbindungsabschnitt 4 sich an der Nase der Person abstützt und sich die Schenkel 3a, 3b in Richtung der Ohren erstrecken. In dem Tragbügel 2 sind mehrere Applikatoren gelagert, wobei in dem Verbindungsabschnitt 4 ein Paar von Augenapplikatoren und an den Schenkeln 3a, 3b jeweils ein Ohrenapplikator 5a, 5b gelagert sind. Der jeweilige Applikator umfasst eine Spule 6 zur Erzeugung eines magnetischen Feldes bei einem Stromfluss durch die Spule 6, wobei das Paar von Augenapplikatoren zur Behandlung der Augen der Person dient und die Ohrenapplikatoren 5a, 5b zur Behandlung der Ohren der Person dienen, und die Spulen 6 zu diesem Zweck dazu eingerichtet sind, in dem entsprechenden Bereich ein Magnetfeld zu applizieren. Bei den Spulen 6 handelt es sich jeweils um einen gewickelten Leiter in Form eines Kupferdrahts, wobei die Spulen 6 der Augenapplikatoren jeweils 100 Wicklungen und die Spulen 6 der Ohrenapplikatoren 5a, 5b jeweils 60 Wicklungen aufweisen.

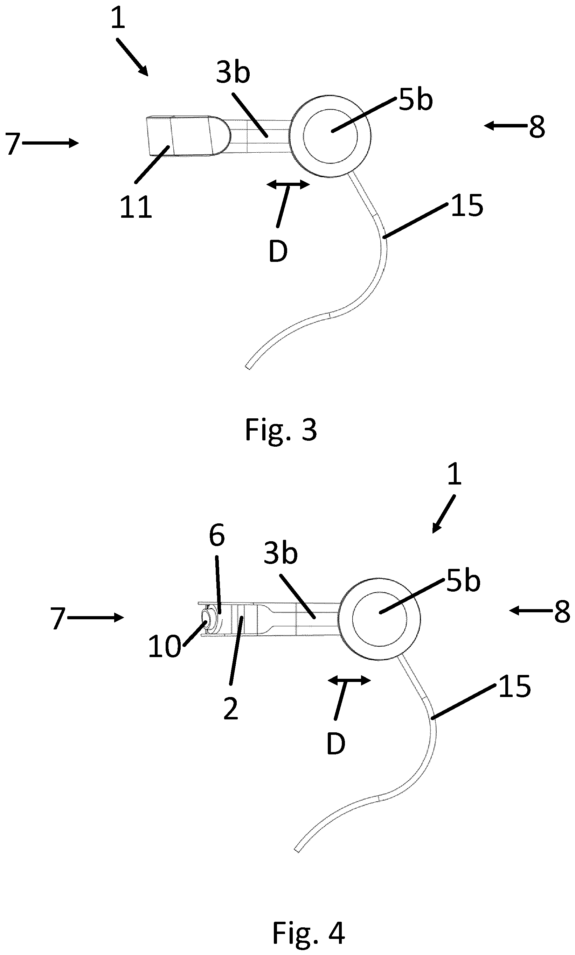

Die Schenkel 3a, 3b sind einteilig mit dem Verbindungsabschnitt 4 ausgebildet, wie insbesondere der Fig. 2 zu entnehmen ist. Im Bereich des Verbindungsabschnitts 4 sind die Spulen 6 der Augenapplikatoren ortsfest angeordnet, wohingegen der erste Ohrenapplikator 5a und der zweite Ohrenapplikator 5b im Bereich eines freien Endes des jeweiligen Schenkels 3a, 3b entlang des jeweiligen Schenkels 3a, 3b linear verschiebbar und somit ortsveränderlich ist. Diese Verschiebbarkeit ist durch den Doppelpfeil D in den Fig. 3 und 4 kenntlich gemacht. Durch dieses Gestaltung kann eine einfache Anpassung der Applikatoreinrichtung 1 an die jeweiligen Anatomie des Kopfes der zu behandelnden Person erfolgen.

Wie insbesondere den Fig. 2 und 4 zu entnehmen ist, weist der Verbindungsabschnitt 4 zwei in Richtung einer der Person abzuwendenden Frontseite 7 hervorstehende, ringförmige Vorsprünge auf, wobei der jeweilige Vorsprung einen hohlzylindrischen Lagerabschnitt 10 bildet, auf den die ringförmige Spule 6 des jeweiligen Augenapplikators frontseitig aufgeschoben oder herumgewickelt ist. Der jeweilige Lagerabschnitt 10 weist weder einen Boden noch einen Deckel auf, wodurch sich eine Sichtöffnung 9 für das jeweilige Auge ergibt. Dadurch ist es der die Applikatoreinrichtung 1 tragenden Person möglich, durch die Applikatoreinrichtung 1 nach außen zu schauen.

An der der Person abgewandten Frontseite 7 der Applikatoreinrichtung 1 ist ein transparentes, frontseitig verspiegeltes Verkleidungselement 11 in Form eines U-förmigen Visiers angebracht. Da das Verkleidungselement 11 transparent ist, ist es trotz der Tatsache, dass dieses Verkleidungselement 11 den Verbindungsabschnitt und somit auch die Sichtöffnungen 9 vollständig und die beiden Schenkel 3a, 3b teilweise abdeckt, der die Applikatoreinrichtung 1 tragenden Person dennoch möglich, nach draußen zu blicken.

Das Verkleidungselement 11 ist formstabiler ausgebildet als der Tragbügel 2, wobei aufgrund der Tatsache, dass das Verkleidungselement 11 lediglich im Bereich des Verbindungsabschnitts 4 an dem Tragbügel 2 befestigt ist, die Schenkel 3a, 3b dennoch verformt werden können. Dabei ist eine Verformung der Schenkel 3a, 3b nach außen, somit voneinander weg, durch die mit den Schenkeln 3a, 3b überlappenden Abschnitten des Verkleidungselements 11 beschränkt. Nach innen hingegen, somit aufeinander zu, ist ein Verformen der Schenkel 3a, 3b nicht durch das Verkleidungselement 11 beschränkt, sodass lediglich eine maximale Öffnungsweite des Tragbügels 2 durch das Verkleidungselement 11 beschränkt wird.

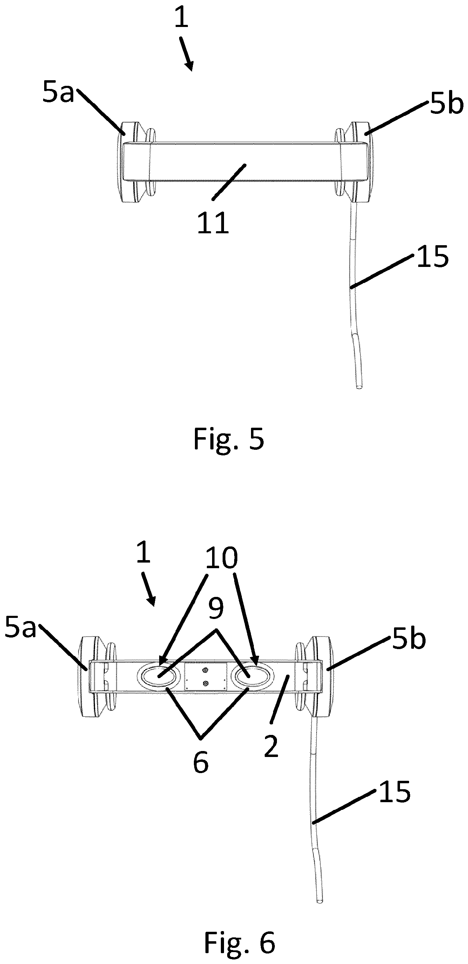

Die Applikatoreinrichtung 1 weist eine gemeinsame erste Signalerzeugungseinheit 12 für die Augenapplikatoren auf, wobei die gemeinsame erste Signalerzeugungseinheit 12 mit den Spulen 6 der Augenapplikatoren elektrisch verbunden ist, wobei die gemeinsame erste Signalerzeugungseinheit 12 dazu eingerichtet ist, dass die Spulen 6 der Augenapplikatoren durchfließende Stromsignal zu erzeugen, wobei die gemeinsame erste Signalerzeugungseinheit 12 in dem linken Ohrenapplikator 5b integriert ist. Diese Gestaltung ist in der Fig. 5 näher dargestellt. Die gemeinsame erste Signalerzeugungseinheit 12 ist durch eine Leiterplatte 13 mit daran angeordneten Elektronikkomponenten 14 gebildet. Wie man der Fig. 5 entnehmen kann, ist im Bereich des linken Ohrenapplikators 5a auch ein Anschlusskabel 15 vorgesehen, das dem Verbinden der Applikatoreinrichtung 1 mit einer externen Stromversorgung, insbesondere einer Gleichstromquelle, dient. Die elektrische Verbindung der ersten Signalerzeugungseinheit 12 mit den Spulen 6 der Augenapplikatoren erfolgt mittels eines Flachbandkabels, wobei dieses Flachbandkabel in einem innenliegenden Kabelkanal 7 des Tragbügels 2 geführt ist, der sich von dem linken Schenkel 3b bis zu dem Verbindungsabschnitt 4 erstreckt. Ein entsprechender Kabelkanal 7 erstreckt sich von dem rechten Schenkel 3a bis zu dem Verbindungsabschnitt 4.

In dem rechten Ohrenapplikator 5a ist eine zweite gemeinsame Signalerzeugungseinheit angeordnet, wobei diese zweite Signalerzeugungseinheit mit den Spulen 6 des jeweiligen Ohrenapplikators 5a, 5b elektrisch verbunden ist, wobei die zweite Signalerzeugungseinheit dazu eingerichtet ist, dass die Spulen 6 der Ohrenapplikatoren 5a, 5b durchfließende Stromsignal zu erzeugen, wobei der linke Ohrenapplikator 5b wiederum über eine Verkabelung, die in dem Tragbügel 2 geführt ist, mit der zweiten, in dem ersten Ohrenapplikator 5a ausgebildeten gemeinsamen zweiten Signalerzeugungseinheit elektrisch verbunden ist. Diese zweite Signalerzeugungseinheit weist wiederum eine Leiterplatte mit daran angebrachten Elektronickomponenten auf.

Die Leiterplatte der jeweiligen Signalerzeugungseinheit 12 ist kreisförmig ausgebildet und somit an die geometrische Gestaltung eines Gehäuses des jeweiligen Ohrenapplikators 5a, 5b angepasst. Das jeweilige Gehäuse ist zweiteilig ausgebildet und weist eine äußere Gehäuseschale 16 und eine innere Gehäuseschale 17 auf. An der inneren, dem Ohr der zu behandelnden Person zugewandten Gehäuseschale 17 ist ein Schaumstoffelement 18 angebracht, um den Tragekomfort zu erhöhen.

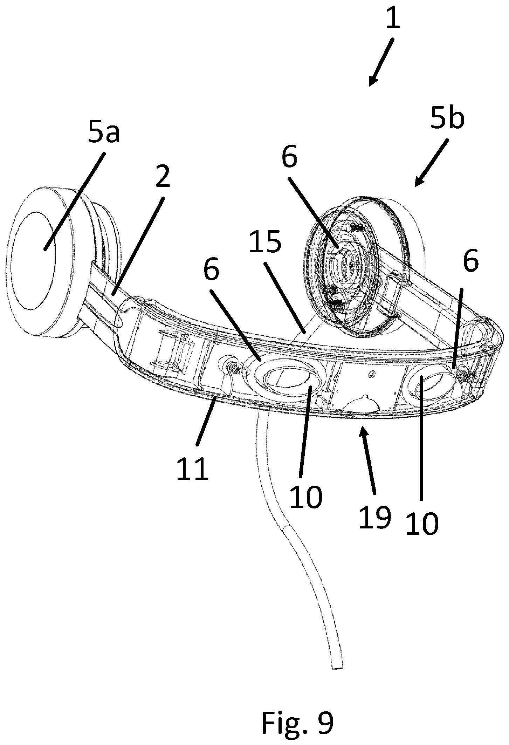

Die Fig. 9 zeigt eine zweite Ausführungsform der Applikatoreinrichtung 1, die sich von der ersten Ausführungsform im Wesentlichen durch die Gestaltung des Verbindungsabschnitts 4 und des Verkleidungselements 11 unterscheidet. Bei der zweiten Ausführungsform ist im Gegensatz zu der ersten Ausführungsform sowohl an dem Verbindungsabschnitt 4 als auch an dem Verkleidungselement 11 eine Aussparung 19 für eine Nase der Person vorgesehen.

### Bezugszeichenliste

- 1: Applikatoreinrichtung
- 2: Tragbügel
- 3a: erster Schenkel
- 3b: zweiter Schenkel
- 4: Verbindungsabschnitt
- 5a: erster Ohrenapplikator
- 5b: zweiter Ohrenapplikator
- 6: Spule
- 7: Frontseite
- 8: Rückseite
- 9: Sichtöffnung
- 10: Lagerabschnitt
- 11: Verkleidungselement
- 12: erste Signalerzeugungseinheit
- 13: Leiterplatte
- 14: Elektronikkomponente
- 15: Anschlusskabel
- 16: äußere Gehäuseschale
- 17: innere Gehäuseschale
- 18: Schaumstoffelement
- 19: Aussparung

## Patentansprüche

1. Applikatoreinrichtung (1) zum Applizieren von magnetischen Feldern zur Magnetfeldtherapie, wobei die Applikatoreinrichtung (1) einen elastisch verformbaren Tragbügel (2) mit einem ersten Schenkel (3a), einem zweite Schenkel (3b) und mit einem den ersten Schenkel (3a) mit dem zweiten Schenkel (3b) verbindenden Verbindungsabschnitt (4) aufweist, wobei der Tragbügel (2) dazu angepasst ist, bei Verwendung der Applikatoreinrichtung (1) im Kopfbereich einer Person getragen zu werden, derart, dass der Verbindungsabschnitt (4) sich über das Gesicht der Person, insbesondere die Augen der Person erstreckt, und sich die Schenkel (3a, 3b) in Richtung der Ohren erstrecken, wobei in oder an dem Verbindungsabschnitt (4) ein Paar von Augenapplikatoren und in oder an den Schenkeln (3a, 3b) jeweils ein Ohrenapplikator (5a, 5b) angeordnet sind, wobei der jeweilige Applikator eine Spule (6) aufweist zur Erzeugung eines magnetischen Feldes bei einem Stromfluss durch die Spule (6).

2. Applikatoreinrichtung (1) nach Anspruch 1, wobei der Tragbügel (2) U-förmig gebogen ausgebildet ist, wobei im Bereich des Verbindungsabschnitts (4) die Spulen (6) der Augenapplikatoren ortsfest angeordnet sind und der erste Ohrenapplikator (5a) im Bereich eines freien Endes des ersten Schenkels (3a) und der zweite Ohrenapplikator (5b) im Bereich eines freien Endes des zweiten Schenkels (3b) angeordnet sind.

3. Applikatoreinrichtung (1) nach Anspruch 1 oder 2, wobei der jeweilige Ohrenapplikator (5a, 5b) entlang des jeweiligen Schenkels (3a, 3b) verschiebbar an dem jeweiligen Schenkel (3a, 3b) gelagert ist.

4. Applikatoreinrichtung (1) nach einem der Ansprüche 1 bis 3, wobei die Schenkel (3a, 3b) einteilig mit dem Verbindungsabschnitt (4) ausgebildet sind.

5. Applikatoreinrichtung (1) nach einem der Ansprüche 1 bis 4, wobei der jeweilige Ohrenapplikator (5a, 5b) ein Gehäuse umfasst, wobei in dem Gehäuse die Spule (6) des jeweiligen Ohrenapplikators (5a, 5b) aufgenommen ist.

6. Applikatoreinrichtung (1) nach einem der Ansprüche 1 bis 5, wobei der Verbindungsabschnitt (4) eine Sichtöffnung (9) für das jeweilige Auge aufweist, vorzugweise die dem jeweiligen Auge zugeordnete Spule (6) die dem jeweiligen Auge zugeordnete Sichtöffnung (9) umlaufend umschließt.

7. Applikatoreinrichtung (1) nach einem der Ansprüche 1 bis 6, wobei der Verbindunssabschnitt (4) einen dem jeweiligen Augenapplikator zugeordneten, hohlzylindrischen Lagerabschnitt (10) aufweist, wobei die Spule (6) des jeweiligen Augenapplikators auf den Lagerabschnitt (10) aufgeschoben oder herumgewickelt ist zum radialen Lagern der Spule (6).

8. Applikatoreinrichtung (1) nach einem der Ansprüche 1 bis 7, wobei an dem Verbindungsabschnitt (4) auf einer der Person abzuwenden Frontseite (7) ein den Verbindungsabschnitt (4) abdeckendes Verkleidungselement (11) angebracht ist, wobei das Verkleidungselement (11) an dem Verbindungsabschnitt (4) befestigt ist, derart, dass die Schenkel (3a, 3b) unabhängig von dem Verkleidungselement (4) elastisch verformbar sind.

9. Applikatoreinrichtung (1) nach Anspruch 8, wobei das Verkleidungselement (11) lösbar an dem Verbindungsabschnitt (4) angebracht ist.

10. Applikatoreinrichtung (1) nach einem der Ansprüche 1 bis 9, wobei das Verkleidungselement (11) formstabiler ist als der Tragbügel (2).

11. Applikatoreinrichtung (1) nach einem der Ansprüche 1 bis 10, wobei das Verkleidungselement (11) zumindest im Bereich der Sichtöffnungen (9) durchsichtig/transparent ist.

12. Applikatoreinrichtung (1) nach einem der Ansprüche 1 bis 11, wobei die Applikatoreinrichtung (1) eine erste Signalerzeugungseinheit (12) für den jeweiligen Augenapplikator oder eine gemeinsame erste Signalerzeugungseinheit (12) für beide Augenapplikatoren aufweist, wobei die ersten Signalerzeugungseinheiten oder die gemeinsame erste Signalerzeugungseinheit (12) mit den Spulen (6) der Augenapplikatoren elektrisch verbunden sind, wobei die ersten Signalerzeugungseinheiten (12) oder die gemeinsame erste Signalerzeugungseinheit (12) dazu eingerichtet sind, das die Spulen (6) der Augenapplikatoren durchfließende Stromsignal zu erzeugen, wobei die ersten Signalerzeugungseinheiten (12) oder die gemeinsame erste Signalerzeugungseinheit (12) in einem der Ohrenapplikatoren (5a, 5b) integriert sind.

13. Applikatoreinrichtung (1) nach einem der Ansprüche 1 bis 12, wobei die Applikatoreinrichtung (1) eine zweite Signalerzeugungseinheit für den jeweiligen Ohrenapplikator (5a, 5b) oder eine gemeinsame zweite Signalerzeugungseinheit für beide Ohrenapplikatoren (5a, 5b) aufweist, wobei die zweiten Signalerzeugungseinheiten oder die gemeinsame zweite Signalerzeugungseinheit mit den Spulen (6) der Ohrenapplikatoren (5a, 5b) elektrisch verbunden sind, wobei die zweiten Signalerzeugungseinheiten oder die gemeinsame zweite Signalerzeugungseinheit dazu eingerichtet sind, das die Spulen (6) der Ohrenapplikatoren (5a, 5b) durchfließende Stromsignal zu erzeugen, wobei die zweiten Signalerzeugungseinheiten oder die gemeinsame zweite Signalerzeugungseinheit in einem der Ohrenapplikatoren (5a, 5b) integriert sind.

14. Applikatoreinrichtung (1) nach einem der Ansprüche 1 bis 13, wobei in dem jeweiligen Ohrenapplikator (5a, 5b) zumindest eine der Signalerzeugungseinheiten (12) integriert ist, insbesondere in dem einen Ohrenapplikator (5a, 5b) die gemeinsame erste Signalerzeugungseinheit (12) und in dem anderen Ohrenapplikator (5a, 5b) die gemeinsame zweite Signalerzeugungseinheit integriert ist.

15. Applikatoreinrichtung (1) nach einem der Ansprüche 12 bis 14, die jeweilige Signalerzeugungseinheit (12) eine Leiterplatte (13) mit darauf angebrachten Elektronikkomponenten (14) umfasst.
